Europäisches Patentamt

European Patent Office

Office européen des brevets

(19)

(11) **EP 1 053 715 B1**

(12) **EUROPEAN PATENT SPECIFICATION**

(45) Date of publication and mention
of the grant of the patent:
**30.06.2004 Bulletin 2004/27**

(21) Application number: **98928676.0**

(22) Date of filing: **24.04.1998**

(51) Int Cl.[7]: **A61B 5/05**, G01R 27/00

(86) International application number:
**PCT/RU1998/000123**

(87) International publication number:
**WO 1998/048693 (05.11.1998 Gazette 1998/44)**

(54) **METHOD FOR MEASURING THE ELECTRICAL CONDUCTION OF ORGANIC TISSUES**

VERFAHREN ZUR MESSUNG DER ELEKTRISCHEN LEITFÄHIGKEIT VON ORGANGEWEBE

PROCEDE DE MESURE DE LA CONDUCTIVITE ELECTRIQUE DE TISSUS ORGANIQUES

(84) Designated Contracting States:
**AT CH DE ES FR GB IT LI NL SE**

(30) Priority: **29.04.1997 RU 97107063**

(43) Date of publication of application:
**22.11.2000 Bulletin 2000/47**

(73) Proprietor: **Karasev, Alexandr Alexandrovich
Taganrog, 34939 (RU)**

(72) Inventor: **Karasev, Alexandr Alexandrovich
Taganrog, 34939 (RU)**

(74) Representative: **Beetz & Partner Patentanwälte
Steinsdorfstrasse 10
80538 München (DE)**

(56) References cited:
**EP-A- 0 747 005        WO-A-94/15529
DE-C- 950 402          SU-A- 707 573
SU-A- 785 740          US-A- 4 160 447**

## Description

## TECHNICAL FIELD

The invention relates to the medicine and can be used in various fields of science and engineering that require information about the components of the complex impedance of the biological object's tissue.

## BACKGROUND OF THE INVENTION

Lately, the trends of scientific and diagnostic studies of biological objects that make provision for analysis of parameters of measured complex impedance of various body areas of a biological object, so-called impedance methods, are being - more and more widely practised. The main point of these methods is to measure the electrical parameters of the skin of a biological object in various body areas and compare them with the already studied parameters of the standard skin sample. Having determined to what extend these parameters deviate from the standard, a conclusion can be drawn about skin condition, diseases and other phenomena in a living organism.

Known in the art is a method for measuring the skin resistance protected by the author's certificate of USSR №1.821195 INT.CL. A61H 39/00, A61B 5/05, published in 1993 EM №22, that involves the following: the measuring electrodes are put onto the skin, stabilized DC impulses of 200...380 milliseconds duration under current density of 7.1...36. $2\mu A$ are conducted between the electrodes, the resistance at the end of every impulse is repeatedly measured, the correction value to the measured resistance is calculated as difference between the resistance value measured for the first time and the resistance value measured for the second time (in 42 seconds after the first measuring), and the resistance value of every next measuring is determined taking into account this correction.

The disadvantage of this method is that the results of measurements are affected by external factors, such as, starting or stopping of perspiration, the way the electrodes are pressed to the skin, concentration of electrolyte in the corneous layer. That is why, this method provides low diagnostic potentialities as the results of measurements cannot be repeated.

The method for measuring the skin conductivity in the area of biologically active points (see description to author's certificate of USSR N! 1801472 INT.CL. A61H 39/02 published in 1993, БИ N!10) makes it possible to reduce measurement error at the expense of spread of resistance of the tissue between the electrodes. According to this method, the cap of the active electrode is filled up with cotton fabric, impregnated with electrolyte. The stabilized constant voltage source of 12 V is connected to the electrodes through variable resistor, active and passive electrodes are strapped and the value of short circuit current of 200 (A is set with the help of the variable resistor, then, the passive electrode is clasped tightly in the right hand and the active electrode is put to the biologically active point under examination, after 4-5 seconds the current is measured and the skin conductivity in the area of biologically active point is calculated. This method also provides low diagnostic potentialities, as the results of measurements cannot be repeated in consequence of affection of external factors.

According to the method for measuring the electrical resistance of biological objects with two electrodes, protected by the author's certificate of USSR N!1204182 INT.CL.4 A61B 5/05, G01R 27/02, published in 1986, БИ N! 2, the electrodes arc put upon the biological object under examination, the measuring current is conducted between the electrodes and the resistance R1 of the tissue between the electrodes is measured, then the value of measuring current is changed and the area of the electrodes k fold, provided that external dimensions of the electrodes are not changed and the new value of resistance R2 of the tissue between the electrodes is measured, the value of the resistance $R_T$ of the biological object's tissue and the resistance $R_3$ of the tissue under the electrodes are calculated from the formulas:

$$R_1 = \frac{kR_1 - R_2}{k-1},$$

$$R_0 = \frac{R_2 - R_1}{k-1},$$

This method is of little use when applied for measuring conductivity of tissue of a living organism, as it can cause burns and other disoders of electrophysiological condition of the tissue. Moreover, it makes it impossible to measure the capacitive component of the complex impedance of the biological object's tissue.

Known in the art are methods for measuring the resistive and capacitive components by analyzing transient processes when there is jump-in of voltage across the resistance being measured.

According to the method for measuring the electrical quantity of resistance, inductance and capacitance, protected by the author's certificate of USSR № 1797079 INT.CL. G01R 27/26 published in 1993, БИ №7, the DC voltage is applied to series active-capacitive or active-inductive measuring circuit with one of its components known, the first instantaneous value of voltage is measured at the midpoint of the measuring circuit in a standard time interval since the moment of applying voltage at the midpoint of measuring circuit, in a time interval, such as above, since the moment of the first measuring, then the second instantaneous value of voltage is measured at the midpoint of the measuring circuit and the unknown component is determined from the formulas:

- for active-capacitive circuit,

$$C_x = - \frac{\Delta t}{R_0 \ln \frac{U_2 - U_1'}{U_1}},$$

$$R_x = - \frac{\Delta t}{C_0 \ln \frac{U_2 - U_1'}{U_1}},$$

- for inductive-active circuit.

$$L_x = - \frac{R_0 \Delta t}{\ln \frac{U_2 - U_1'}{U_1}},$$

$$R_x = - \frac{L_0 \ln \frac{U_2 - U_1}{U_1}}{\Delta t},$$

Where

Cx, Rx, Lx - are the values of the unknown component of the measuring circuit,
Co, Ro, Lo - are the values of the known component of the measuring circuit,
(t - is a standard time interval,
U1, U2 - are the first and the second instantaneous values of voltage at the midpoint of the measuring circuit.

The disadvantage of this method is that it does not provide quite accurate measurement of values of active and reactive components of the complex impedance. Moreover, it cannot be applied for measuring the conductivity of the biological object's tissue because of low accuracy, as Lhe results of measurement are to a great extend affected by instability of resistance of the tissue, both, its resistive and capacitive components.
In the description to the author's certificate of USSR №17075696 INT.CL. G01R 27/18 published in 1992, БИ N!3, there described the method for measuring the resistive and the capacitive components of the complex impedance, according to which the resistance being measured is periodically connected, first, to a standard voltage source for the a priori preset time $t_1$, then, the resistance being measured is short-circuited, the instantaneous value of a voltage drop across the resistance is measured at the end of the time interval $t_1$ and in the a priori preset time $t_1 = t_2$ after short-circuiting. The resistive and the capacitive components are calculated by the formulas:

$$R_x = R_n \left[ \frac{\ln\left(1 - \frac{U_1}{U_2}\right)}{\ln \frac{U_2}{U_1}} - 1 \right],$$

$$C_X = - \frac{t_1}{R_0 \left[ \ln\left( 1 - \frac{U_1}{U_0} \right) - \ln\frac{U_2}{U_1} \right]},$$

Where Ro - is the differential resistance of the standard voltage source,

$U_o$ - is the voltage of the standard power supply,
$U_1$ - is the instantaneous value of voltage across the complex impedance at the end of the time interval t1,
$U_2$ - is the instantaneous value of voltage at the end of the time interval t2 = t1after short-circuiting,
$t_1 = t_2$ - is a priory preset time interval.

The disadvantage of this method is that it provides low accuracy, as the results of measurement are to a great extend affected by the resistance of the tissue under the electrodes.

Resonance methods of measuring the components of the complex impedance make it possible to reduce the effect of the resistance under the electrodes upon the results of measurement. Thus, using the resonance method for measuring the capacitance, described in the book of R.G. Karpov and N.P. Karpov "Electroradiomeasurement" (published in Moscow, "High school", in 1978, see pages 140 -141, Fig. 7.13), or the resonance the resistance, described ibid. at page 142, figure 7.17, the complex impedance of the biological object's tissue can be measured with high accuracy. According to this method, radio frequency oscillator is connected by loose coupling with the oscillatory circuit that consists of known inductance I, and - changed capacitance Cnep, that have the capacitance or the resistance being measured connected in series to them. The oscillatory circuit is adjusted to resonance, then the measured resistance is short-circuited and the change of the variable capacitor provides the oscillatory circuit being readjusted. The value of the capacitance to be found is determined by the difference in two values of the variable capacitor capacitance.

The disadvantage of the resonance methods is that measuring takes much time, as to do this requires the components of the oscillatory circuit to be changed and the resonance to be found out.

Known in the art are methods of non-contact measurement of specific electrical resistance of semiconductor films.

The method for measuring the components of the complex impedance that is put into practice by the device, patented in Russia (patent N! 2003123, INT.CL. G01R 27/26 published in 1993, БИ N!41-42), is the closest by its nature to the applied one. This method consists in the following. The complex impedance to be measured is connected in parallel to measuring oscillatory circuit (or connected in series to series oscillatory circuit) of the self-excited oscillator supplied with frequency-independent bridge rectifier and the reactive component of the complex impedance is determined from variation of frequency of self-excited oscillations, the resistive component is determined from the amplitude output of the bridge rectifier.

If applied to measuring the conductivity of the biological object's tissue, this method of measurement provides simultaneous determination of resistive and capacitive components of the complex impedance of the tissue between the electrodes.

Prior art described in patent N! 2003128, INT.CL. G01R 27/26, published in 1993, БИ №41-42, has following features coinciding with the applied method:

- the measuring electrodes are put onto the biological object's skin area under examination;
- the components of the complex impedance between the electrodes are determined by parameters of electrical oscillations in the oscillatory circuit, including as its component the complex impedance between the electrodes.

The disadvantage of the prior art lies in its low accuracy because of instability of frequency of self-excited oscillator and the amplitude of these oscillations being affected by the resistance of skin under the electrodes.

The aim of the invention is to increase the accuracy of measuring the conductivity of the biological object's tissue.

EP0747005 discloses a method for measuring a patient's impedance by using a plurality of electrodes. It uses a signal generator constantly outputting a fixed frequency AC signal to tissues, and then measuring the out-coming voltage between the electrodes.

## SUMMARY OF THE INVENTION

The above mentioned aim is achieved by the fact that in the method for measuring the conductivity of the biological object's tissue that consists in superimposing the electrodes upon the skin area under examination and determining the components of the complex impedance of the conductivity of the biological object's tissue by parameters of electrical oscillations in the oscillatory circuit, including as its component the complex impedance of the tissue between the electrodes, the conductance coil preliminarily saturated with electromagnetic energy is connected to the electrodes and the components of the biological object's tissue are determined by the parameters of free oscillations that set on in the oscillatory circuit, including as its components the inductance of the inductance coil, the ohmic resistance and the capacitance of the biological object's tissue.

The inductance coil is saturated with electromagnetic energy in doses by means of connecting the conductance coil to the constant voltage source for the a priori preset time.

The resistive component of the inductance of the biological object's tissue is determined by the rate of damping of free oscillations in the oscillatory circuit.

The capacitive component of the inductance of the biological object's tissue is determined by the amplitude of the first semiwave of oscillations of voltage between the electrodes after the conductance coil saturated with electromagnetic energy has been connected to the electrodes.

The ohmic resistance and the electrostatic capacity of the tissue between the electrodes is calculated by the expressions:

$$R_x = -\frac{2L\ln\frac{U_2}{U_1}}{T},$$

$$C_x = L\left[\frac{U_0}{(r_0 + r_L)U_m}\left(1 - e^{-\frac{r_0 + r_L}{L}\tau}\right)\right]^2,$$

Where $R_x$ - is the ohmic resistance of the tissue between the electrodes;

$C_x$ - is the electrostatic capacity of the tissue between the electrodes;
L - is the inductance of the inductance coil;
$r_o$ - is the internal resistance of the constant voltage source;
$r_L$ - is the ohmic resistance of the inductance coil;
T - is the time interval for measuring the rate of damping - of free oscillations in the oscillatory circuit;
$\tau$ - is the time interval for the conductance coil being connected to constant voltage source;
$U_1$- is the amplitude of oscillations at the beginning of the time interval T;
$U_2$- is the amplitude of oscillations at the end of the time interval T;
$U_o$ - is the voltage of the constant voltage source;
$U_m$ - is the amplitude of the first semiwave of oscillations of voltage between the electrodes after the conductance coil saturated with electromagnetic energy has been connected to the electrodes.

The feature of the invention, i.e. the connection of saturated with electromagnetic energy inductance coil to the electrodes placed upon the biological object's skin area under examination, in order to determine the components of the complex impedance of the biological object's tissue, is unknown from the available prior art. Determining of the components of the complex impedance by parameters of free oscillations in the oscillatory circuit, having the inductance of the inductance coil saturated with electromagnetic energy and the impedance of the biological object's tissue between the electrodes is unknown as well.

**BRIEF DESCRIPTION OF THE DRAWINGS**

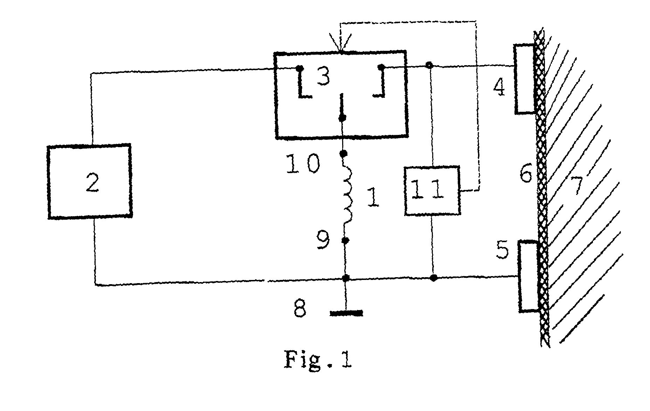

The essence of the applied method is revealed by the description of operation of the device (for the functional diagram of the device see Fig.1) that provides the applied method for measuring the conductivity of the biological object's tissue being put into practice.

The device consists of high-quality conductance coil 1, standard source of constant voltage 2, switch 3, active electrode 4 and passive electrode 5, that are put upon the biological object's skin area 6 under examination, in order to measure the conductivity of the tissue 7 between the electrodes. The passive electrode 5 is connected to a common bus 8, that, in its turn, has one of the terminals of the source 2 and the lead of the conductance coil 1 being connected to it. The unit of control and measurement connected to the tap of the conductance coil 1 provides the measurement of the parameters of free oscillations in the oscillatory circuit.

**REALIZATION OF INVENTION**

To measure the conductivity of the biological object's tissue 7, the tap 10 of the conductance coil 1 is connected through the switch 3, controlled by the unit 11 to the source 2, for a priori preset time interval. The electromagnetic energy, accumulated in the conductance coil 1 during the time interval $\tau$, can be calculated by expression:

$$\Theta = LI_\tau^2 = L\left[\frac{U_0}{r_0 + r_L}\left(1 - e^{-\frac{r_0 + r_L}{L}\tau}\right)\right]^2 \quad (1),$$

Where

L - is the inductance of the inductance coil 1;
I$\tau$ - is the current conducted through the conductance coil 1 at the end of the time interval $\tau$;
$U_o$ - is the voltage of the source 2;
$r_o$ - is the internal resistance of the source 2;
$r_L$ - is the ohmic resistance of the conductance coil 1;

By changing the time interval $\tau$ the quality of electromagnetic energy, accumulated in the conductance coil 1, can be changed.
The formula (1) is correct with permissible accuracy for the inductance coil, having high quality of 500 and more, inter-turn capacitance of the conductance coil may be ignored.
When the time interval $\tau$ comes to an end, the lead 10 of the conductance coil 1 is disconnected with the help of the switch 3 from the source 2 and connected to the active electrode 4.
As the resistance of the tissue 7 is capacitive in its effect, the connection of the lead 10 of the conductance coil 1 to the electrode 4 produces a series oscillatory circuit that, in its turn, provides the onset of free oscillations. This oscillatory circuit has as its components: the inductance of the inductance coil 1, contact resistance "active electrode 4 - skin 6", parallel connection of the resistance of the skin area 6 between the electrodes with the resistance of hypodermic tissue 7 between the electrodes, contact resistance "passive electrode 5 - skin 6".
Free oscillations that set on in this oscillatory circuit will change in a following manner:

$$U(t) = U_m e^{\alpha\tau}\cos(\omega t) \quad (2),$$

Where

$U_m$ - is the peak amplitude of oscillations of voltage between the electrodes that is equal to the amplitude of the first semiwave;
$\alpha$ - is the rate of damping of oscillations;
$\omega = 2\pi f$ - is the angular oscillation frequency.

The peak amplitude of free oscillations of voltage between the electrodes 4, 5 (the amplitude of the first semiwave) depends on the quantity of electromagnetic energy accumulated in the conductance coil 1 and is proportional to the peak current in the conductance coil 1 that is equal to It - the current at the end of the time interval $\tau$.

$$U_m = \sqrt{\frac{L}{C_\Theta}}I_\tau = \sqrt{\frac{L}{C_\Theta}}\frac{U_0}{r_0 + r_L}\left(1 - e^{-\frac{r_0 + r_L}{L}\tau}\right) \quad (3),$$

- Where, besides the above designation, $C_3$ - is the equivalent capacitance in the oscillatory circuit.
If the values L, $U_o$, $r_o$, $r_L$ and $\tau$ are known (they can be measured with high accuracy beforehand), the measurement of the amplitude of the first semiwave of oscillations $U_m$ provides the equivalent capacitance being calculated from the expression (3) by the formula:

$$C_э = L\left[\frac{U_0}{(r_0 + r_L)U_m}\left(1 - e^{-\frac{r_0+r_L}{L}T}\right)\right]^2 \quad (4).$$

The rate of damping of free oscillations in the oscillatory circuit can be determined by measuring the amplitude of oscillations at the beginning and at the end of the time interval, that is multiple of the period of oscillations, by the formula:

$$\alpha = -\frac{\ln\left(\frac{U_2}{U_1}\right)}{T}, \quad (5)$$

Where

$\alpha = \frac{R_э}{2L}$ - is the rate of damping of free oscillations;

$U_1$ - is the amplitude of free oscillations at the beginning of the time interval T;

$R_э$ - is the equivalent ohmic resistance of the oscillatory circuit;

$T \geq \frac{10}{2\pi f}$ - is the time interval for the rate of damping of the oscillations being measured;

$f$ - is the frequency of free oscillations.

Using the formula ( 5 ) and taking into account that $\alpha = \frac{R_3}{2L}$, the equivalent ohmic resistance R is determined by the expression:

$$R_э = -\frac{2L\ln\left(\frac{U_2}{U_1}\right)}{T} \quad (6).$$

The frequency of free oscillations in oscillatory circuit is determined by the expression:

$$f = \frac{1}{2\pi}\sqrt{\left(\frac{L}{C_э} - \frac{R_э}{4L^2}\right)} \quad (7).$$

Considering, that the ohmic resistance of the inductance coil 1 is equal to several ohms, the ohmic resistance of the tissue 7 between the electrodes is equal to 200-300Ω, the ohmic resistance of the skin area 6 between the electrodes is equal to several hundreds of kOhm and the the capacitance of transition "electrode - biological object's tissue" is two orders more than the capacitance of the tissue 7 between the electrodes, having the area of electrodes more than 0.2 cm$^2$, it can be ascertained that, if the frequency of free oscillations is $f \geq 100$ kHz, the ohmic and the capacitive components of the complex impedance of the biological object's tissue 7 are equal to the values determined from the formulas (4), (5), (6) with the error not more than 5 %.

Thus, if the high-quality conductance coil is saturated with electromagnetic energy in doses and then immediately connected to the electrodes put upon the biological object's skin area, the oscillatory circuit formed by the inductance of the inductance coil and the complex impedance of the biological object's tissue between the electrodes will provide the onset of free oscillations of electric current, by the parameters of the the components of the complex impedance (of conductivity) of the biological object's tissue latter can be determined simultaneously. In this case, the results of measurement of parameters of oscillations by the unit 11 practically will not depend either on environment, or nn condition of skin.

The electromagnetic energy, accumulated by the conductance coil, can be dosed with high accuracy by connecting the conductance coil to the constant voltage source for the a priori preset time. The resistive component of the complex impedance of the biological object's tissue can be determined by measuring the rate of damping of free oscillations. The reactive component of the complex impedance of the biological object's tissue can be determined by measuring the amplitude of the first semiwave of oscillations of voltage between the electrodes. By the frequency of free oscillations one can judge about either reactive, or resistive components of the complex impedance of the biological object's tissue.

As compared with the already existing methods of measuring the conductivity of the biological object's tissue, the applied method is characterized by its simplicity, higher accuracy and high diagnostic potentialities. All methods, based on measurements employing direct current or single-polar current provide low accuracy caused by the instability of the resistance of the tissue under the electrodes. The methods of measurement employing the changeable radio-frequency oscillator or audio-frequency oscillator provide lower accuracy, as compared with the applied method in consequence of additional error when determining the resonance. The methods of measurement employing the self-excited oscillator with the oscillatory circuit in a feedback circuit provide lower accuracy, as the resistance of the tissue under the electrodes affects the factor of feedback transmission.

Another advantage of the applied method is that it rules out the possibility of tissue between the electrodes being injured, as the action signal energy is strictly dosed and does not depend on the condition of the skin. Moreover, this signal provides regulation of psychosomatic homeostasis and can be used when treating the various kinds of pathology employing the method of reflexotherapy and at the same time provides the monitoring of the effectiveness of treatment.

## INDUSRIAL APPLICABILITY

The method for measuring the conductivity of the biological object's tissue in the first place can be applied in diagnostics of functional condition of the human organism by analyzing the values of electrical resistance of skin in the area of projections of the internals. Moreover, this method can be used in operation of the electrostimulating devices supplied with the system of monitoring of the action, for example, in devices that determine the action signal level by the reaction of the organism to the action.

The device that puts the applied method into practice can be easily made of the components available in industry. Any stabilized power source with batteries of 1.0-1.5V can be used as a power supply, microprocessor of INTEL type, series 051, can be used as measuring unit, the electrical switch of bipolar transistor of KT817 type can be used as the switch, КИ G22-1.4 can be used as the inductance coil.

## Claims

1. Method for measuring the conductivity of the biological object's tissue consisting in superimposing the electrodes upon the skin area under examination and determining the components of complex impedance of the conductivity of the tissue between the electrodes by the parameters of electric oscillations in oscillatory circuit, including as its component the complex impedance of the biological object's tissue, **characterized in that**, the high-quality conductance coil preliminarily saturated with electromagnetic energy is connected to the electrodes and the components of the conductivity of the biological object's tissue are determined by the parameters of free oscillations that set on in oscillatory circuit, including as its components the inductance of the inductance coil, connected to the electrodes, and the complex impedance of the biological object's tissue between the electrodes.

2. Method as claimed in Claim 1, **characterized in that**, the conductance coil is saturated with electromagnetic energy in doses by means of connecting the conductance coil to constant voltage source for a priory preset time.

3. Method as claimed in Claim 1 or 2, **characterized in that**, the resistive component of the conductivity of the biological object's tissue is determined by the rate of damping of free oscillations in the oscillatory circuit.

4. Method as claimed in Claim 1, 2 or 3, **characterized in that**, the capacitive component of the conductivity of the biological object's tissue is determined by the amplitude of the first semiwave of oscillations of voltage between the electrodes after the conductance coil, saturated with electromagnetic energy, has been connected to the electrodes.

5. Method according to the one of the Claims 1, 2, 3 or 4, **characterized in that**, the ohmic resistance and the capacitance of the tissue between the electrodes are determined by the following expressions:

$$R_x = -\frac{2\,L\ln\frac{U_2}{U_1}}{T},$$

$$C_x = L\left[\frac{U_0}{(r_0 + r_L)U_m}\left(1 - e^{-\frac{r_0 + r_L}{L}\tau}\right)\right]^2,$$

Where

$R_x$ -is the ohmic resistance of the tissue between the electrodes;
$C_x$ - is the electrostatic capacity of the tissue between the electrodes;
L - is the inductance of the inductance coil;
$r_0$- is the internal resistance of constant voltage source;
$r_L$ - is the ohmic resistance of the inductance coil;
T - is the time interval for measuring the rate of damping of free oscillations;
$U_1$- is the amplitude of oscillations at the beginning of the time interval T;
$U_2$- is the amplitude of oscillations at the end of the time interval T;
$\tau$- is the time interval for the conductance coil being connected to constant voltage source;
$U_0$ - is the voltage of the constant voltage source;
$U_m$ - is the peak amplitude (the amplitude of the first semiwave) of oscillations of voltage between the electrodes after the conductance coil saturated with electromagnetic energy has been connected to the electrodes.

**Patentansprüche**

1. Verfahren zur Messung der elektrischen Leitfähigkeit von Gewebe eines biologischen Objekts, das darin besteht, die Elektroden auf den zu untersuchenden Bereich der Haut aufzulegen und die Komponenten der Kompleximpedanz der elektrischen Leitfähigkeit des Gewebes zwischen den Elektroden durch die Parameter der elektrischen Schwingungen in einem Schwingkreis zu bestimmen, der als Komponente die Kompleximpedanz des Gewebes des biologischen Objekts aufweist, **dadurch gekennzeichnet, dass** die vorläufig mit elektromagnetischer Energie gesättigte hochwertige Konduktanzspule mit den Elektroden verbunden wird, und die Komponenten der elektrischen Leitfähigkeit des Gewebes des biologischen Objekts durch die Parameter von freien Schwingungen bestimmt werden, die im Schwingkreis entstehen, der als Komponenten die Induktivität der mit den Elektroden verbundene Drosselspule und die Kompleximpedanz des Gewebes des biologischen Objekts zwischen den Elektroden enthält.

2. Verfahren nach Anspruch 1, **dadurch gekennzeichnet, dass** die Konduktanzspule durch Verbinden der Konduktanzspule mit der Konstantspannungsquelle über einen vorher festgelegten Zeitraum dosisweise mit elektromagnetischer Energie gesättigt wird.

3. Verfahren nach Anspruch 1 oder 2, **dadurch gekennzeichnet, dass** die Widerstandskomponente der elektrischen Leitfähigkeit des Gewebes des biologischen Objekts durch die Dämpfungsrate der freien Schwingungen im Schwingkreis bestimmt wird.

4. Verfahren nach einem der Ansprüche 1, 2 oder 3, **dadurch gekennzeichnet, dass** die kapazitive Komponente der elektrischen Leitfähigkeit des Gewebes des biologischen Objekts durch die Amplitude der ersten Halbwelle der Spannungsschwingungen zwischen den Elektroden bestimmt wird, nachdem die mit elektromagnetischer Energie gesättigte Konduktanzspule mit den Elektroden verbunden wurde.

5. Verfahren nach einem der Ansprüche 1, 2, 3 oder 4, **dadurch gekennzeichnet, dass** der ohmsche Widerstand und die elektrische Kapazität des Gewebes zwischen den Elektroden durch die folgenden Ausdrücke bestimmt werden:

$$R_x = -\frac{2L\ln\frac{U_2}{U_1}}{T},$$

$$C_X = L\left[\frac{U_0}{(r_0 + r_L)U_m}\left(1 - e^{-\frac{r_0+r_L}{L}\tau}\right)\right]^2,$$

wobei

$R_x$      der ohmsche Widerstand des Gewebes zwischen den Elektroden ist;
$C_x$      die elektrostatische Kapazität des Gewebes zwischen den Elektroden ist;
L        die Induktivität der Drosselspule ist;
$R_o$      der innere Widerstand der Konstantspannungsquelle ist;
$r_L$      der ohmsche Widerstand der Drosselspule ist;
T        das Zeitintervall zum Messen der Dämpfungsrate der freien Schwingungen ist;
$U_1$      die Amplitude der Schwingungen zu Beginn des Zeitintervalls T ist;
$U_2$      die Amplitude der Schwingungen am Ende des Zeitintervalls T ist;
$\tau$        das Zeitintervall für die Verbindung der Konduktanzspule mit der Konstantspannungsquelle ist;
$U_o$      die Spannung der Konstantspannungsquelle ist;
Um      die höchste Amplitude (die Amplitude der ersten Halbwelle) der Spannungsschwingungen zwischen den Elektroden ist, nachdem die mit elektromagnetischer Energie gesättigte Konduktanzspule mit den Elektroden verbunden wurde.

**Revendications**

1. Procédé de mesure de la conductivité du tissu d'un objet biologique, qui consiste à appliquer les électrodes sur la zone de la peau à examiner et à déterminer les composantes d'impédance complexe de la conductivité du tissu entre les électrodes par les paramètres des oscillations électriques dans un circuit oscillant, comportant en tant que composante l'impédance complexe du tissu de l'objet biologique, **caractérisé en ce que** la bobine de conductance de haute qualité présaturée d'énergie électromagnétique est connectée aux électrodes et que les composantes de la conductivité du tissu de l'objet biologique sont déterminées par les paramètres d'oscillations libres émanant du circuit oscillant, comprenant en tant que composantes l'inductance de la bobine d'inductance connectée aux électrodes et l'impédance complexe du tissu de l'objet biologique entre les électrodes.

2. Procédé selon la revendication 1, **caractérisé en ce que** la bobine de conductance est saturée d'énergie électromagnétique par doses par l'intermédiaire de sa connexion à la source de tension continue pendant un intervalle de temps défini au préalable.

3. Procédé selon la revendication 1 ou 2, **caractérisé en ce que** la composante résistive de la conductivité du tissu de l'objet biologique est déterminée par l'indice d'amortissement des oscillations libres dans le circuit oscillant.

4. Procédé selon l'une des revendications 1, 2 ou 3, **caractérisé en ce que** la composante capacitive de la conductivité du tissu de l'objet biologique est déterminée par l'amplitude de la première demi-onde d'oscillations de tension entre les électrodes après la connexion de la bobine de conductance saturée d'énergie électromagnétique aux électrodes.

5. Procédé selon l'une des revendications 1, 2, 3 ou 4, **caractérisé en ce que** la résistance ohmique et la capacitance du tissu entre les électrodes sont déterminées selon l'une des expressions suivantes:

$$R_x = -\frac{2L\ln\frac{U_2}{U_1}}{T},$$

$$C_X = L\left[\frac{U_0}{(r_0 + r_L)U_m}\left(1 - e^{-\frac{r_0 + r_L}{L}\tau}\right)\right]^2,$$

où

$R_x$     est la résistance ohmique du tissu entre les électrodes;
$C_x$     est la capacité électrostatique du tissu entre les électrodes;
L     est l'inductance de la bobine d'inductance;
$r_o$     est la résistance interne de la source de tension continue;
$r_L$     est la résistance ohmique de la bobine d'inductance;
T     est l'intervalle de temps pour mesurer l'indice d'amortissement des oscillations libres;
$U_1$     est l'amplitude des oscillations au début de l'intervalle de temps T;
$U_2$     est l'amplitude des oscillations à la fin de l'intervalle de temps T;
$\tau$     est l'intervalle de temps pour la connexion de la bobine de conductance à la source de tension continue;
$U_o$     est la tension de la source de tension continue;
$U_m$     est l'amplitude maximale (l'amplitude de la première demi-onde) des oscillations de tension entre les électrodes après la connexion à la bobine de conductance saturée d'énergie électromagnétique de ces derniè-res.

Fig.1